# EUROPEAN PATENT APPLICATION

(11) **EP 1 408 039 A2**
(43) Date of publication of application: **14.04.2004**
(21) Application number: 03078795.6
(22) Date of filing: 30.11.1999
(51) Int. Cl.: C07D 405/12, A61K 31/445

(54) **Mixed paraxetine propan-2-ol solvates**

(30) Priority: 30.11.1998 GB 9826182; 12.07.1999 GB 9916257
(62) Divisional of application: 99973027.8
(71) Applicant: SMITHKLINE BEECHAM PLC, Brentford, Middlesex TW8 9GS (GB)
(72) Inventor: Craig, Andrew Simon, Tonbridge, Kent TN11 9AN (GB); Jacewicz, Victor Witold, Tonbridge, Kent TN11 9AN (GB); Ward, Neal, Tonbridge, Kent TN11 9AN (GB)
(74) Representative: Knight, Lucie Viktoria

(57) **Abstract**

A crystalline paroxetine hydrochloride mixed solvate, that is more easily desolvated than the propan-2-ol solvate to form the anhydrate, is obtained by contacting crystalline propan-2-ol solvate with a transformation solvent. Suitable transformation solvents are an ether, ketone, chlorinated hydrocarbon, nitrile, lower alcohol, or ester, or a mixture thereof.

Crystalline paroxetine hydrochloride anhydrate is obtained by heating the mixed solvate in a vacuum oven.

## Description

The present invention relates to a process for the preparation of a pharmaceutically active compound and intermediates thereof, and to the use of the active compound in therapy. In particular this invention is concerned with a new process for the preparation of a mixed paroxetine chloride solvate and its use to prepare a crystalline anhydrate form of paroxetine hydrochloride.

Pharmaceutical products with antidepressant and anti-Parkinson properties are described in US-A-3912743 and US-A-4007196. An especially important compound among those disclosed is paroxetine, the (-)*trans* isomer of 4-(4'-fluorophenyl)-3-(3',4'-methylenedioxyphenoxymethyl)-piperidine. This compound is used in therapy as the hydrochloride salt for the treatment and prophylaxis of *inter alia* depression, obsessive compulsive disorder (OCD) and panic.

Paroxetine hydrochloride has been described in the literature as a crystalline hemihydrate (see EP-A-0223403 of Beecham Group) and as various crystalline anhydrate forms (see WO 96/24595 of SmithKline Beecham). WO 96/24595 describes the preparation of paroxetine hydrochloride propan-2-ol solvate which is a useful intermediate for the preparation of paroxetine hydrochloride anhydrate Form A. When prepared conventionally by crystallisation from anhydrous propan-2-ol, paroxetine hydrochloride propan-2-ol solvate has very poor stirring properties, and is very difficult to isolate, wash, and desolvate.

The desolvation of paroxetine hydrochloride propan-2-ol solvate has been described in WO 96/25495 and EP 0 812 827 A1. It is clear from these publications that desolvation in a vacuum oven is a slow and difficult process. Furthermore, when operated on a scale larger than a few grammes, the time and temperature necessary for substantially complete desolvation increase very greatly, for example EP 0 812 827 A1 states that on a 180g scale drying for 1 week at 80°C gives a product still containing more than 2% propan-2-ol, and that the temperature must be increased to 110°C before the propan-2-ol residue can be reduced to approximately 0.1%. Lengthy treatment is undesirable for manufacturing because of the costs associated with vessel occupancy, and high temperatures cause degradation and phase transformation in the product. Another undesirable feature of the existing process is the tendency for the product to crystallise in a heavy matted form which is very difficult to stir, transfer, and filter. For example, a glass-lined reactor containing a vigorously stirred solution of 20 kg of paroxetine hydrochloride in 160 litres of propan-2-ol was seeded at 48°C. The entire contents set to a thick crystalline mass, and it was necessary to open the man-way and manually dislodge the contents so that the mixture could be stirred and the product collected. It is therefore evident that the existing process is unsuitable for the large scale manufacture of paroxetine hydrochloride.

This invention provides processes for the preparation of a modified paroxetine hydrochloride propan-2-ol solvate which is more easily desolvated than paroxetine hydrochloride propan-2-ol solvate prepared in a conventional manner, and hence more suitable for commercial manufacturing processes.

According to one aspect of the present invention there is provided as a novel compound a crystalline paroxetine hydrochloride which is solvated with propan-2-ol and at least one other solvent.

Especially the present invention provides crystalline paroxetine hydrochloride acetone propan-2-ol mixed solvate.

According to another aspect of the present invention there is provided a process for preparing a crystalline paroxetine hydrochloride mixed solvate which comprises contacting crystalline paroxetine hydrochloride propan-2-ol solvate with a transformation solvent.

The crystalline paroxetine hydrochloride propan-2-ol solvate is typically prepared by providing a solution of paroxetine hydrochloride in propan-2-ol, and crystallising the propan-2-ol solvate from the solution. Alternatively, the crystalline paroxetine hydrochloride propan-2-ol solvate may be prepared by adding propan-2-ol to paroxetine hydrochloride, which may be in crystalline or non-crystalline form (e.g. an amorphous solid or an oil), and stirring.

In a further aspect of the invention, the mixed solvate may be prepared by crystallising paroxetine hydrochloride from a solution in propan-2-ol and a transformation solvent.

The transformation solvent is a solvent which results in the form of the crystalline product being changed to a mixed solvate, which is more easily desolvated. The transformation solvent may be an ether, ketone, chlorinated hydrocarbon, nitrile, lower alcohol, or ester, or a mixture of two or more of these solvents, for example diethyl ether, tetrahydrofuran, acetone, butanone, chloroform, dichloromethane, acetonitrile, methanol, or ethyl acetate. A particularly suitable solvent is a mixture of acetone and methanol or acetone alone.

A suitable concentration of paroxetine hydrochloride in propan-2-ol for the initial crystallisation is one part in between 4 and 40 volumes of propan-2-ol, preferably between 8 and 20 volumes, and more preferably between 10 and 14 volumes. Preferably the propan-2-ol is anhydrous to suppress formation of paroxetine hydrochloride hemihydrate. Crystallisation may be initiated by conventional procedures such as cooling a heated solution, or removing solvent by evaporation or heating. It may be advantageous to add seeds of crystalline paroxetine hydrochloride propan-2-ol solvate prepared by the procedure of this invention or by the procedure of WO 96/24595.

Alternatively, the transformation solvent may be added to paroxetine hydrochloride propan-2-ol solvate crystals as a slurry in propan-2-ol, or after partial or complete removal of the propan-2-ol mother liquor. The resulting slurry is suitably contacted with the transformation solvent for at least 20 minutes, preferably with efficient stirring, more preferably for 2 hours or longer. The treatment may be repeated a number of times. For example, using acetone the treatment may be carried out at least two times for three hours in between 3 and 8 volumes of acetone, preferably between 4 and 5 volumes of acetone.

In another embodiment of the invention, the treatment with transformation solvent is carried out by a slow perfusion through a packed cake of paroxetine hydrochloride propan-2-ol solvate. This may be carried out conveniently on a manufacturing scale in, for example, a filter drier.

During the treatment with the transformation solvent, a proportion of the paroxetine hydrochloride may dissolve. This loss is greater at higher temperatures, but the rate of transformation to mixed solvate is advantageously increased. The loss in yield may be minimised by cooling before final isolation. Consequently, an advantageous implementation of this invention is to carry out the transformation at elevated temperature and to cool before isolation. This procedure is, in effect, a partial or at the extreme, a complete recrystallisation from mixed solvent.

In a particular aspect of the invention, paroxetine hydrochloride with a bound organic solvent content of less than 1% may be prepared by desolvating the mixed solvate by heating at a temperature not exceeding 70°C in 48 hours or less.

A surprising result of the processes of this invention is that, under comparable desolvation conditions, the product contains less residual organic solvent than is obtained by desolvating the propan-2-ol solvate. This is especially noticable in the case of the mixed propan-2-ol:acetone solvate when compared to the properties of either the pure propan-2-ol solvate or the pure acetone solvate.

Suitable procedures for preparing paroxetine hydrochloride for dissolution in propan-2-ol for use in the process of this invention include those mentioned in US Patents 4,009,196; 4,721,723; 4,902,801; 4,861,893 and 5,039,803.

The crystalline paroxetine hydrochloride propan-2-ol mixed solvates obtainable by the process of this invention are a different crystalline form from the previously known individual solvates.

It has been found that paroxetine hydrochloride propan-2-ol mixed solvates may be processed and desolvated more easily than conventionally produced paroxetine hydrochloride propan-2-ol solvate.

The crystallisation process may include if necessary isolating the propan-2-ol solvate crystals, optionally washing and drying. However advantageously the crystalline propan-2-ol solvate is not dried, but used as solvent wet cake as a feed material for further processing to obtain a paroxetine hydrochloride anhydrate, especially the Form A anhydrate.

Accordingly, in a further aspect the present invention provides a process for preparing a crystalline anhydrate of paroxetine hydrochloride by heating a crystalline propan-2-ol mixed solvate of this invention to remove bound propan-2-ol and the transformation solvent.

Desolvation is conveniently carried out by heating in an oven, preferably *in vacuo,* suitably at a temperature of about 60°C.

The resultant anhydrate desirably contains less than 2% of propan-2-ol, preferably less than 1%, more preferably less than 0.5%, and most preferably less than 0.1%.

The crystalline paroxetine hydrochloride anhydrate obtainable by this invention may be used in therapy in formulations described in EP-A-0223403 or WO 96/00477.

Therapeutic uses of the paroxetine hydrochloride anhydrate of this invention include treatment of *inter alia* : alcoholism, anxiety, depression, obsessive compulsive disorder (OCD), panic disorder, chronic pain, obesity, senile dementia, migraine, bulimia, anorexia, social phobia, pre-menstrual syndrome (PMS), adolescent depression, trichotillomania, dysthymia, and substance abuse, referred to below as "the Disorders".

Most suitably the anhydrate obtainable by the present invention is applied to the treatment of depression, OCD and panic.

The compositions prepared in accordance with this invention are usually adapted for oral administration, but formulations for dissolution for parental administration are also within the scope of this invention.

The composition is usually presented as a unit dose composition containing from 1 to 200mg of active ingredient calculated on a free base basis, more usually from 5 to 100 mg, for example 10 to 50 mg such as 10, 12.5, 15, 20, 25, 30 or 40 mg by a human patient.

Most preferably unit doses contain 20 mg of active ingredient calculated on a free base basis. Such a composition is normally taken from 1 to 6 times daily, for example 2, 3 or 4 times daily so that the total amount of active agent administered is within the range 5 to 400 mg of active ingredient calculated on a free base basis. Most preferably the unit dose is taken once a day.

Preferred unit dosage forms include tablets or capsules, including formulations adapted for controlled or delayed release.

The compositions of this invention may be formulated by conventional methods of admixture such as blending, filling and compressing. Suitable carriers for use in this invention include a diluent, a binder, a disintegrant, a colouring agent, a flavouring agent and/or preservative. These agents may be utilized in conventional manner, for example in a manner similar to that already used for marketed anti-depressant agents.

Specific examples of pharmaceutical compositions include those described EP-B-0223403, and US 4,007,196 in which the anhydrate product of the present invention may be used as the active ingredient.

Accordingly, the present invention also provides:
a pharmaceutical composition for treatment or prophylaxis of the Disorders comprising paroxetine hydrochloride anhydrate obtainable by this invention and a pharmaceutically acceptable carrier;
the use of paroxetine hydrochloride anhydrate obtainable by this invention to manufacture a medicament for the treatment or prophylaxis of the Disorders; and
a method of treating the disorders which comprises administering an effective or prophylactic amount of paroxetine hydrochloride anhydrate obtainable by this invention to a person suffering from one or more of the Disorders.

The invention is illustrated by the following Examples. In the Examples and Reference Examples desolvating has been carried out under standardised conditions of apparatus and temperature for the purpose of comparison. In the Examples, greater desolvation is achieved in less time than in the Reference Examples. Paroxetine hydrochloride with lower residual propan-2-ol can be obtained by increasing the heating time or temperature.

### Reference Example 1 ( propan-2-ol solvate)

A hot solution of paroxetine hydrochloride (12g ) in propan-2-ol ( 200 ml ) was stirred under a nitrogen atmosphere and then allowed to cool to ambient temperature. After 4 hours the crystalline product was collected by filtration. This material was desolvated by heating under vacuum at 60°C for 64 hours. Yield 11.1g (propan-2-ol content 5.6%).

### Reference Example 2 ( acetone solvate)

A hot solution of paroxetine hydrochloride (10 g) in acetone (450 ml) was stirred under a nitrogen atmosphere and then allowed to cool to ambient temperature. The solution was allowed to crystallize overnight, then the product was collected by filtration, and desolvated by heating under vacuum at 60°C for 64 hours. Yield 9.1 g (acetone content 2.2%).

### Example 1

Paroxetine hydrochloride propan-2-ol solvate crystals (34.0g) were stirred in acetone (120 ml) under a nitrogen atmosphere for 3 hours. The solid was isolated by filtration and desolvated at 60°C under vacuum for 42 hours. Yield 32 g ( propan-2-ol content 1.45%, acetone content 0.5%).

### Example 2

The desolvated paroxetine hydrochloride product from Example 3 (15 g) was stirred in acetone (60 ml) under a nitrogen atmosphere for 3 hours. The solid was isolated by filtration and desolvated at 60°C under vacuum for 42 hours. Yield 14.1 g ( propan-2-ol content 0.1 %, acetone content 0.7 %).

### Example 3

A solution ofparoxetine hydrochloride (5.0 g) in propan-2-ol (70 ml) and acetone (10 ml) was heated at reflux for 20 minutes under nitrogen. The homogeneous solution was then cooled to room temperature under argon without stirring. Once crystallisation had started, the mixture was stirred and propan-2-ol was added (30 ml). The product was collected by filtration, washed with propan-2-ol (15 ml) and desolvated in vacuo for 20 hours at 60°C to give paroxetine hydrochloride anhydrate Form A (4.26 g) containing 3.5% propan-2-ol and 0.1% acetone by weight.

### Example 4

Acetonitrile (60 ml) was added to paroxetine hydrochloride propan-2-ol solvate crystals (10.0 g) and the mixture stirred as a slurry at ambient temperature under a nitrogen atmosphere for 2 hours. The solid was collected by vacuum filtration and desolvated by heating under vacuum at 60°C for 40 hours. Yield 8.7 g (propan-2-ol content 0.2%, acetonitrile content 0.3 %).

### Example 5

Tetrahydrofuran (5.0 ml) was added to paroxetine hydrochloride propan-2-ol solvate crystals (1.0 g) and the mixture stirred as a slurry at ambient temperature under a nitrogen atmosphere for 2 hours. The solid was collected by vacuum filtration and desolvated by heating under vacuum at 60°C for 40 hours. Yield 0.92 g (propan-2-ol content 0.1 %, tetrahydrofuran content 1.7% ).

### Example 6

Dichloromethane (40 ml) was added to paroxetine hydrochloride propan-2-ol solvate crystals (10.0 g) and the mixture stirred as a slurry at ambient temperature under a nitrogen atmosphere for 2 hours. The solid was collected by vacuum filtration and desolvated by heating under vacuum at 60°C for 40 hours. Yield 6.3 g (propan-2-ol content 0.4%, dichloromethane content 2.1 %).

### Example 7

Chloroform (5.0 ml) was added to paroxetine hydrochloride propan-2-ol solvate crystals (0.98 g) and the mixture stirred as a slurry at ambient temperature under a nitrogen atmosphere for 2 hours. The solid was collected by vacuum filtration and desolvated by heating under vacuum at 60°C for 40 hours. Yield 0.78 g (propan-2-ol content 0.1%, chloroform content 2.7%).

### Example 8

A mixture of acetone (75 ml) and methanol (5 ml) was added to paroxetine hydrochloride propan-2-ol solvate crystals (5.0 g) and the mixture stirred as a slurry at ambient temperature under a nitrogen atmosphere for 16 hours. The slurry was cooled to 5°C and stirred for a further 30 minutes. The product was collected by vacuum filtration and desolvated by heating under vacuum at 60°C for 40 hours. Yield 3.5 g (propan-2-ol content less than 0.1 %, acetone content 0.1 %).

### Example 9

Methanol (5 ml) was added to paroxetine hydrochloride propan-2-ol solvate crystals (5.0 g) and the mixture stirred at ambient temperature under a nitrogen atmosphere for 10 minutes to produce a clear solution. Acetone (50 ml) was added and the stirring continued, whereupon a thick suspension was formed. After stirring for 10 minutes the product was collected by vacuum filtration and desolvated by heating under vacuum at 60°C for 20 hours. Yield 3.0 g (propan-2-ol content less than 0.1 %, acetone content 0.1 %).

### Example 10

Acetone (200 ml) was added to paroxetine hydrochloride propan-2-ol solvate crystals (5.0 g) under a nitrogen atmosphere and the mixture stirred and heated to reflux for 10 minutes. The resulting solution was allowed to cool to ambient temperature and was stirred at this temperature for 1 hour. The crystalline product was collected by vacuum filtration and desolvated by heating at 60°C under vacuum for 1 hour to produce paroxetine hydrochloride anhydrate containing 2.1 % acetone and 0.1 % propan-2-ol. The acetone content was reduced to 1.0% by heating at 60°C under vacuum for a further 19 hours. Yield 4.10 g.

### Example 11

A stirred mixture of N-phenoxycarbonyl paroxetine (19.4 g), potassium hydroxide (17.5 g) and toluene (300 ml) was heated to reflux under a nitrogen atmosphere for 3 hours. The mixture was cooled to room temperature, washed with water (200 ml) and the organic layer separated, dried over magnesium sulphate and concentrated to an oil (14.0 g). Propan-2-ol (90 ml) was added to the oil and the mixture stirred under a nitrogen atmosphere until a complete solution was observed. A solution of hydrogen chloride in propan-2-ol [5-6 N] (9.0 ml) was added and the mixture stirred for 30 minutes, and then the product was collected by vacuum filtration. Acetone (150 ml) was added to the solvent-wet filter cake and the mixture stirred and heated to reflux under a nitrogen atmosphere for 5 minutes. The solution was allowed to cool to room temperature and the resulting crystalline mixed solvate collected by vacuum filtration. The product was desolvated under vacuum at ambient temperature for 16 hours. Yield 11.1 g (propan-2-ol content 1.0%, acetone content 2.9%).

The product was further desolvated by heating under vacuum at 60°C for 20 hours to produce paroxetine hydrochloride containing propan-2-ol (0.7%) and acetone (1.3%).

### Example 12

A solution of paroxetine hydrochloride (17.0 kg) in propan-2-ol (137 L) and glacial acetic acid (0.275 kg) was heated to reflux in a 50 gallon glass-lined reactor, held at reflux for 15 minutes, and cooled to 70°C. n-Hexane (52 L) and finely powdered seed crystals of paroxetine hydrochloride propan-2-ol solvate (ca 17 g) were added and the well-stirred mixture allowed to crystallise at 60-65 °C for 40 minutes. The contents of the reactor were then cooled to about 25 °C and stirred for a further 2 hours.

The white crystals were transferred under nitrogen to a Guedu filter drier and washed with hexane (2 x 33.5 L). Acetone (126 L) was allowed to permeate slowly through the filter cake over a period of 4 hours. The product was then desolvated under vacuum (ca 30 mbar) in the filter drier at 35 - 40°C for 11 hours, agitating for 5 minutes each hour.

A sample taken at this point was analysed for solvent content by NMR and found to contain 8.8% propan-2-ol and 4.9% acetone.

The solvated paroxetine hydrochloride was desolvated by increasing the drying temperature to 60°C over a period of 11 hours, and continuing the vacuum drying at 60 - 70 °C for a further 13 hours with constant agitation.

The resulting paroxetine hydrochloride anhydrate was found to contain 0.8% propan-2-ol.
The acetone content was less than 0.1 %.

## Claims

1. A process for obtaining a crystalline paroxetine hydrochloride mixed solvate which comprises contacting crystalline paroxetine hydrochloride propan-2-ol solvate with a transformation solvent, in which the transformation solvent is an ether, ketone, chlorinated hydrocarbon, nitrile, lower alcohol, or ester, or a mixture thereof.

2. A process according to claim 1 where the transformation solvent is diethyl ether, tetrahydrofuran, acetone, butanone, chloroform, dichloromethane, acetonitrile, methanol, or ethyl acetate.

3. A process according to claim 2 where the transformation solvent is acetone or a mixture of acetone and methanol.

4. A process according to any one of claims 1 to 3 where the transformation solvent is added to a slurry of paroxetine hydrochloride propan-2-ol solvate crystals after partial or complete removal of the propan-2-ol mother liquor.

5. A process according to any one of claims 1 to 4 where the paroxetine hydrochloride propan-2-ol solvate crystals are contacted with the transformation solvent for between 20 minutes and 2 hours with efficient stirring.

6. A process according to any one of claims 1 to 5 where the process is carried out between 2 and 5 times.

7. A process according to any one of claims 1 to 6 in which the contact with the transformation solvent is carried out by slow perfusion through a packed cake of the solvate.

8. A process according to claim 7 where the slow perfusion is carried out in a filter drier.

9. A process according to any one of claims 1 to 8 in which the contact of paroxetine hydrochloride propan-2-ol solvate with transformation solvent is carried out at elevated temperature, resulting in partial or complete dissolution, followed by cooling to induce crystallisation.

10. A process according to claim 9 where the transformation solvent is acetone.

11. A process according to any one of claims 1 to 10 where the volume of transformation solvent is between 2 and 10 times the volume of the paroxetine hydrochloride propan-2-ol solvate, preferably between 3 and 6 times.

12. A process for preparing a crystalline anhydrate of paroxetine hydrochloride by desolvating a crystalline propan-2-ol mixed solvate obtainable by the process of any one of claims 1 to 11.

13. A process according to claim 12 where the desolvation is carried out by heating at reduced pressure.

14. A process according to claim 13 where the temperature is raised in stages to between 60 and 80°C.

15. A pharmaceutical composition for treatment or prophylaxis of alcoholism, anxiety, depression, obsessive compulsive disorder (OCD), panic disorder, chronic pain, obesity, senile dementia, migraine, bulimia, anorexia, social phobia, pre-menstrual syndrome (PMS), adolescent depression, trichotillomania, dysthymia, and substance abuse comprising paroxetine hydrochloride anhydrate obtainable by the process of any one of claims 12 to 14 and a pharmaceutically acceptable carrier.

16. Use of paroxetine hydrochloride anhydrate obtainable by the process of any one of claims 12 to 14 to manufacture a medicament for the treatment or prophylaxis of alcoholism, anxiety, depression, obsessive compulsive disorder (OCD), panic disorder, chronic pain, obesity, senile dementia, migraine, bulimia, anorexia, social phobia, pre-menstrual syndrome (PMS), adolescent depression, trichotillomania, dysthymia, and substance abuse.
